(19) Europäisches Patentamt
European Patent Office
Office européen des brevets

(11) Veröffentlichungsnummer: **0 514 780 A2**

(12) # EUROPÄISCHE PATENTANMELDUNG

(21) Anmeldenummer: **92108190.7**

(22) Anmeldetag: **15.05.92**

(51) Int. Cl.⁵: **C12P 41/00**, C12P 13/00, C07C 229/12

(30) Priorität: **24.05.91 DE 4116980**

(43) Veröffentlichungstag der Anmeldung: **25.11.92 Patentblatt 92/48**

(84) Benannte Vertragsstaaten: **AT BE CH DE ES FR GB IT LI NL SE**

(71) Anmelder: **Degussa Aktiengesellschaft**
**Weissfrauenstrasse 9**
**W-6000 Frankturt am Main 1(DE)**

(72) Erfinder: **Groeger, Ulrich, Dr.**
**Löherstrasse 39 B**
**W-8750 Aschaffenburg(DE)**
Erfinder: **Drauz, Karlheinz, Dr.**
**Zur Marienruhe 15**
**W-6463 Freigericht 1(DE)**

(54) **Verfahren zur Herstellung enantiomerenreiner offenkettiger N-Alkyl-L oder D-aminosäuren.**

(57) 2.1. Offenkettige enantiomerenreine N-Alkyl-L oder D-aminosäuren wurden bisher chemisch mit erheblichem Reinigungsaufwand, z. B. Chromatographie über chirale Säulen, hergestellt. Das neue Verfahren soll bei guten Ausbeuten und ohne größerem oder teurerem Reinigungsaufwand durchzuführen und auch für die industrielle Produktion geeignet sein.

2.2. Aus einer racemischen Mischung einer offenkettigen N-Acyl-N-alkyl-aminosäure wird mittels einer stereospezifischen auf eine enantiomerenreine N-Acyl-N-alkyl-L oder D-aminosäure abgestimmten Aminoacylase eines der Antipoden zu der entsprechenden enantiomerenreinen offenkettigen N-Alkyl-L oder D-aminosäure gespalten. Anschließend wird entweder die verbliebene Ausgangsverbindung bzw. das Spaltprodukt abgetrennt.

2.3. Herstellung enantiomerenreiner offenkettiger N-Alkyl-aminosäuren für z.B. Cyklosporine.

EP 0 514 780 A2

Die vorliegende Erfindung betrifft ein Verfahren zur Herstellung enantiomerenreiner offenkettiger N-Alkyl-L oder D-aminosäuren und enantiomerenreine N-Alkyl-aminosäuren mit einem freien H oder einer Acylgruppe am N.

Verbindungen dieser Art, vorzugsweise N-Methyl-aminosäuren, sind z. B. Bestandteile der Cyclosporine. Hierbei handelt es sich um zyklische Peptide, die antibiotische und immunsuppressive Wirkung zeigen und aus diesem Grund häufig bei Gewebetransplantationen eingesetzt werden.

Racemate dieser Verbindungen lassen sich in der Regel auf rein chemischem Wege herstellen. Die Trennung dieser Racemate in die einzelnen Enantiomeren ist jedoch sehr aufwendig und muß zum Beispiel über chirale Chromatographiesäulen erfolgen. Ein weiterer Weg zur Herstellung dieser enantiomerenreinen Verbindungen geht über die freien L oder D-Aminosäuren, die dann anschließend an der $\alpha$-Aminogruppe alkyliert und gegebenenfalls acyliert werden.

Hierbei entsteht jedoch eine Vielzahl von Nebenprodukten, die einerseits die Ausbeute erheblich mindern und andererseits oft nur unter großem Aufwand zu entfernen sind.

Eine enzymatische Herstellung dieser Verbindungen, zum Beispiel mittels Acylase scheiterte bisher immer an der zu engen Substratspezifität der verwendeten Acylasen. Die Acylase I ist, wie in Journal of the American Chemical Society 75, 918-920 (1953) und 111, 6354 bis 6364 (1989) beschrieben, geeignet zur Racemattrennung von acylierten $\alpha$-Aminosäuren, sofern diese ein freies Wasserstoffatom in der acylierten Aminogruppe aufweisen. Die Spaltung erfolgt dann L-spezifisch, so daß die N-Acyl-D-aminosäure ungespalten bleibt und zum Beispiel chromatografisch oder durch Ausfällung von der entstandenen L-Aminosäure abgetrennt werden kann. Die Acylase I, die zum Beispiel aus Schweineniere oder aus Aspergillus isoliert werden kann, zeigt zwar eine breite Subtratspezifizität, jedoch keinerlei Aktivität bei N-Acyl-N-alkyl-DL-aminosäuren. In jüngerer Zeit wurden weitere Acylasen gefunden, die eine Spezifität auf endständiges Prolin in Peptiden und auf N-Acylprolin sowie auf vom Prolin abgewandelte ringförmige N-acylierte Aminosäuren haben. Beschrieben werden diese neueren Acylasen in der japanischen Anmeldung 62-232381 (1987), Biochimica et Biophysica Acta, 744 (1983), 180-188 und in EP 0 416 282 A1. Eine Substratspezifität auf offenkettige N-Acyl-N-alkyl-aminosäuren ist von diesen Acylasen nicht bekannt.

Aufgabe der vorliegenden Erfindung ist die Bereitstellung eines Verfahrens zur Herstellung enantiomerenreiner offenkettiger N-Alkyl-L- oder D-aminosäuren, das in guten Ausbeuten und ohne größeren oder teuren Reinigungsaufwand durchzuführen ist und auch für die industrielle Produktion geeignet sein soll.

Diese Aufgabe wird gemäß dem kennzeichnenden Teil des Anspruchs 1 gelöst.

Die Erfindung stellt ein völlig neues Verfahren zur Gewinnung von enantiomerenreinen offenkettigen N-Alkyl-aminosäuren zur Verfügung, wobei diese Aminosäuren, je nach gewünschtem Produkt, auch noch eine N-Acylgruppe aufweisen können. Von Vorteil ist bei dem vorliegenden Verfahren, daß kein Screenen von Bakterienstämmen auf die speziell gewünschte enantiomerenreine N-Alkyl-aminosäure notwendig ist, sondern daß mit leicht zugänglichen N-Acyl-N-alkyl-aminosäuren gescreent werden kann. Wichtig ist hierbei, daß das Screeningsubstrat kein freies H-Atom am N besitzt, wie es meist bei N-acylierten Aminosäuren vorliegt (außer Prolin). Neben den offenkettigen Verbindungen sind hierbei auch die heterozyclischen Verbindungen geeignet, bei denen die eine N-Alkylgruppe direkt oder über ein Heteroatom mit der anderen N-Alkylgruppe, die die Säurefunktion trägt, verbunden ist. Insbesondere kann hier der Umstand ausgenutzt werden, daß mit solchen heterocyclischen Aminosäuren, vorzugsweise Prolin, gescreent werden kann, und daß dann das so erhaltene Enzym zur stereospezifischen Deacylierung der offenkettigen N-Alkyl-L,D-aminosäuren eingesetzbar ist. Da N-acyliertes, zum Screenen vorzugsweise enantiomerenreines Prolin leicht zugänglich und ein geeignetes Screeningsubstrat ist, ermöglicht das erfindungsgemäße Verfahren insgesamt einen neuen und sehr einfachen Weg zur Herstellung der stereospezifischen offenkettigen N-Alkyl-aminosäuren.

Beim Screening wird üblicherweise die chemische Verbindung als alleinige Kohlenstoff- und/oder Stickstoffquelle im Nährmedien eingesetzt, die einer enzymatischen Reaktion zugänglich gemacht werden soll. Zunächst werden "Anreicherungskulturen" angesetzt und auf Mikroorganismen gescreent, die die angebotene chemische Verbindung zum Wachstum nutzen können. Nach Trennung und Reinigung der verschiedenen Mikroorganismen durch gängige mikrobiologische Techniken, werden die so gewonnenen "Reinkulturen" erneut kultiviert, die Zellen gewaschen und aufgeschlossen, z.B. durch mechanische Behandlung mit einer Kugelmühle oder durch Ultraschall. Nach Abtrennung der Zelltrümmer durch Zentrifugation erhält man ein Rohhomogenat, das das gewünschte Enzym enthalten sollte.

Im vorliegenden Fall wurde zur Durchführung des Verfahrens die aus der EP 0 416 282 A1 bekannte N-Acyl-L-prolinacylase verwendet, die aus Comamonas testosteroni DSM 5416 durch Screenen mit N-Acetyl-L-prolin wie in der EP 0 416 282 A1 ausführlich beschrieben erhältlich ist.

Es zeigte sich nun, daß dieses Enzym zur erfindungsgemäßen Herstellung von enantiomerenreinen offenkettigen N-Alkyl-aminosäuren verwendet werden kann und so diese auf einem einfachen Wege

zugänglich macht, obwohl hier auf eine heterocyclische, N-acetylierte Aminosäure gescreent wurde. Von besonderer Bedeutung ist hierbei, daß die Aminoacylase beim Screening nicht auf die entsprechenden offenkettigen N-Acyl-N-alkylaminosäuren abgestimmt sein muß, sondern auch auf N-Acyl-prolin abgestimmt sein kann, ohne daß dabei die Substratsspezifität dann die entsprechenden offenkettigen Aminosäuren ausschließt. Für das erfindungsgemäße Verfahren können also insbesondere bekannte oder durch Screening neu aufgefundene Prolinacylasen eingesetzt werden.

Andererseits können für das erfindungsgemäße Verfahren auch weitere Acylasen eingesetzt werden, die durch Screening mit anderen einfachen Molekülen wie

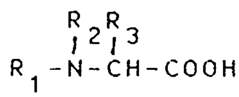

worin

$R^1$ = $COCH_3$ ; $COCH_2Cl$
$R^2$ = $CH_3$ ; $C_2H_5$ ; $C_3H_7$
$R_3$ = H; $CH_3$ ; $CH_3$-CH-$CH_3$; $CH_2$-C($CH_3$)H-$CH_3$;
$CH_3$-CH-$CH_2$-$CH_3$; $CH_2$-OH; HO-CH-$CH_3$;
$CH_2$-SH; $CH_2$-$CH_2$-S-$CH_3$;
$CH_2$-COOH; $CH_2$-$CH_2$-COOH;
$CH_2$-$CONH_2$; $CH_2$-$CH_2$-$CONH_2$;
$CH_2$-$CH_2$-$CH_2$-$CH_2$-$NH_2$;

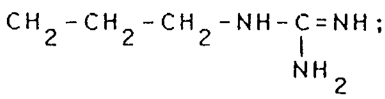

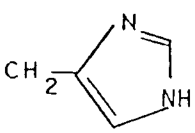 ; 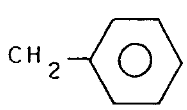 ; 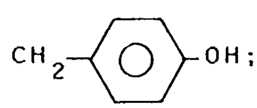

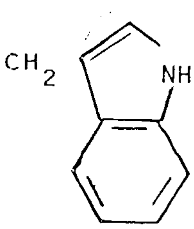

erhältlich sind.

Überlicherweise werden L-spezifische Acylasen zur Durchführung des Verfahrens gesucht und eingesetzt, da diese meist leichter erhältlich sind. Die entsprechenden N-Alkyl-D-aminosäuren sind dann leicht durch chemische Hydrolyse (Deacylierung) der verbliebenen enantiomerenreinen N-Acyl-N-alkyl-D aminosäure erhältlich. Die Deacylierung kann nach literaturbekannten Methoden chemisch im sauren oder alkalischen Milieu unter Retention der Konfiguration zur entsprechenden enantiomerenreinen N-Alkylaminosäure durchgeführt werden.

Das erfindungsgemäße Verfahren wird an Hand nachfolgender Beispiele näher ausgeführt.

Beispiel 1:

Aktivitätsbestimmung der Acylase

Hydrolyse von N-Chloracetyl-N-methyl-L-alanin

1 ml einer 30 mM Lösung von N-Chloracetyl-N-methyl-L-alanin in 0,1 M Tris-HCl, pH 7,0, wurde in ein auf 30°C thermostatisiertes Reaktionsgefäß eingebracht und mit 1,99 ml 0,1 M Tris-HCl, pH 7,0, gemischt. Die Reaktion wurde durch Zugabe von 0,01 ml N-Acyl-L-prolin-acylase aus Comamonas testosteroni DSM 5416 entsprechend 0,23 Units und 0,2 µg Protein gestartet. 1 Unit ist definiert als die Menge an Enzym, die 1µmol N-Acetyl-L-prolin pro Minute bei 30°C und pH 7,0 umsetzt. Zur Erfassung des Reaktionsverlaufes wurden zu verschiedenen Zeitpunkten Proben entnommen, mit 2 N HCl 1:2 versetzt und anschließend durch HPLC unter Verwendung einer RP 8-Säule (250 × 4 mm) analysiert. Nach Elution mit 7 % (v/v) Acetonitril in 0,01 M Heptansulfonsäure (mit 85 %iger $H_3PO_4$ auf pH 2,2 eingestellt) bei einer Flußrate von 2,2 ml/min und einer Säulentemperatur von 40°C wurde die Abnahme von N-Chloracetyl-N-methyl-L-alanin durch Messung der UV-Absorption bei 210 nm detektiert.
Nach einer Reaktionszeit von 60 min betrug der Umsatz 100 %.

Beispiel 2:

Hydrolyse von N-Chloracetyl-N-methyl-D,L-alanin

Es wurde, wie in Beispiel 1 beschrieben, verfahren, jedoch enthielt das Reaktionsgemisch 1 ml einer 60 mM Lösung von N-Chloracetyl-N-methyl-D,L-alanin in 0,1 M Tris-HCl, pH 7,0, 1,98 ml 0,1 M Tris-HCl, pH 7,0 und 0,02 ml Enzym entsprechend 0,46 Units und 0,4 µg Protein. Nach einer Reaktionszeit von 60 min betrug der Umsatz 42 %, nach 120 min betrug der Umsatz 43 %. Die Enantiomerenreinheit des N-Methyl-L-alanins wurde nach Dervatisierung zum N-Trifluoracetylmethylesterderivat zu ≧ 89 % (ee) bestimmt.

Beispiel 3:

Hydrolyse von N-Chloracetyl-N-methyl-D,L-2-aminobuttersäure

Es wurde, wie in Beispiel 1 beschrieben, verfahren, jedoch enthielt das Reaktionsgemisch 1 ml einer 60 mM Lösung von N-Chloracetyl-N-methyl-D,L-2-aminobuttersäure in 0,1 M Tris-HCl, pH 7,0, 1,90 ml 0,1 M Tris-HCl, pH 7,0 und 0,10 ml Enzym entsprechend 2,3 Units und 2 µg Protein. Nach einer Reaktionszeit von 60 min betrug der Umsatz 46 %, nach 120 min betrug der Umsatz 49 %.

Beispiel 4:

Substratspezifität der N-Acyl-L-prolin-Acylase gegenüber verschiedenen N-Acyl-N-alkyl-D,L-aminosäuren

Es wurde, wie in Beispiel 2 und 3 beschrieben, verfahren und die relative Aktivität des Enzyms gegenüber verschiedenen N-Acyl-N-alkyl-D,L-aminosäuren bestimmt. Das Ergebnis zeigt Tabelle 1.

Tabelle 1

| Substrat (20 mM) | Aktivität (%) |
|---|---|
| N-ClAc-N-methyl-D,L-alanin | 100 |
| N-ClAc-N-ethyl-D,L-alanin | 71 |
| N-ClAc-N-propyl-D,L-alanin | 23 |
| N-ClAc-N-methyl-D,L-2-aminobuttersäure | 9 |
| N-ClAc-N-ethyl-D,L-2-aminobuttersäure | 2 |

## Patentansprüche

**1.** Verfahren zur Herstellung enantiomerenreiner offenkettiger N-Alkyl-L oder D-aminosäuren,
dadurch gekennzeichnet,
daß man eine stereospezifische, auf eine enantiomerenreine N-Acyl-N-alkyl-L oder D-aminosäure abgestimmte Aminoacylase auf eine racemische, offenkettige N-Acyl-N-alkyl-aminosäure einwirken läßt und die erhaltene enantiomerenreine offenkettige N-Alkyl-aminosäure oder die verbliebene enantiomerenreine N-Acyl-N-alkylaminosäure abtrennt.

**2.** Verfahren nach Anspruch 1, dadurch gekennzeichnet, daß man eine durch Screenen eines Mikroorganismus mit einer N-Acyl-N-Alkyl-aminosäure erhaltene Aminoacylase einsetzt.

**3.** Verfahren nach Anspruch 2, dadurch gekennzeichnet, daß man eine durch Screenen mit einem einfachen Molekül wie

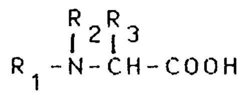

worin

$R^1$ = $COCH_3$; $COCH_2Cl$
$R^2$ = $CH_3$; $C_2H_5$; $C_3H_7$
$R_3$ = H; $CH_3$; $CH_3$-CH-$CH_3$; $CH_2$-C($CH_3$ )H-$CH_3$;
$CH_3$-CH-$CH_2$-$CH_3$; $CH_2$-OH; HO-CH-$CH_3$;
$CH_2$-SH; $CH_2$-$CH_2$-S-$CH_3$;
$CH_2$-COOH; $CH_2$-$CH_2$-COOH;
$CH_2$-$CONH_2$; $CH_2$-$CH_2$-$CONH_2$;
$CH_2$-$CH_2$-$CH_2$-$CH_2$-$NH_2$;

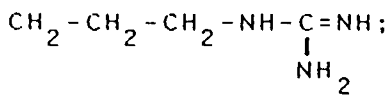

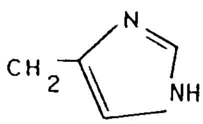 ; 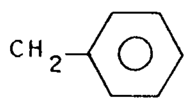 ; 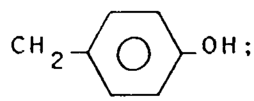

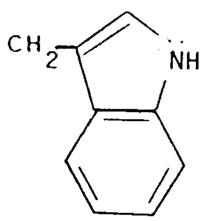 ;

oder $R_2$ und $R_3$ zusammen = $CH_2$-$CH_2$-$CH_2$ erhaltene Aminoacylase einsetzt.

**4.** Verfahren nach Anspruch 2 oder 3, dadurch gekennzeichnet, daß man eine durch Screenen eines Mikroorganismus mit einer enantiomerenreinen N-Acyl-N-alkyl-aminosäure erhaltene Aminoacylase einsetzt.

**5.** Verfahren nach einem der vorhergehenden Ansprüche, dadurch gekennzeichnet, daß man eine stereospezifische N-Acyl-prolinacylase einsetzt.

**6.** Verfahren nach einem der vorhergehenden Ansprüche, dadurch gekennzeichnet, daß man eine L-spezifische Acylase einsetzt.

**7.** Verfahren nach Anspruch 5 oder 6, dadurch gekennzeichnet, daß man eine L-spezifische N-Acyl-prolinacylase aus Comamonas testosteroni DSM 5416 einsetzt.

**8.** Verfahren nach einem der vorhergehenden Ansprüche, dadurch gekennzeichnet, daß man die verbliebene enantiomerenreine N-Acyl-N-alkyl-aminosäure deacyliert.

**9.** Enantiomerenreine N-Alkyl-L oder D-aminosäure mit einem freien H oder einer Acylgruppe am N, hergestellt nach einem der vorhergehenden Ansprüche.

**10.** Enantiomerenreine Verbindung nach Anspruch 9, der allgemeinen Formel

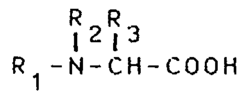

wobei

$R_1$ gleich H; $COCH_3$; $COCH_2Cl$,

$R_2$ gleich $CH_3$; $C_2H_5$; $C_3H_7$

$R_3$ gleich Rest einer natürlichen Aminosäure ist.